# EUROPEAN PATENT SPECIFICATION

(11) **EP 2 853 584 B1**
(45) Date of publication and mention of the grant of the patent: **10.03.2021**
(21) Application number: 13186604.8
(22) Date of filing: 30.09.2013
(51) Int. Cl.: C12M 1/00

(54) **Single-use bioreactor with baffles and a process for manufacturing and operating the same**
Einweg-Bioreaktor mit Stauplatten und Verfahren zur Herstellung und zum Betrieb davon
Bioréacteur à usage unique avec déflecteurs et procédé de fabrication et d'utilisation de ce dernier

(43) Date of publication of application: 01.04.2015
(73) Proprietor: EPPENDORF AG, 22339 Hamburg (DE)
(72) Inventor: Eikelmann, Sven Dipl.-Ing.,, 22299 Hamburg (DE); Selzer, Sebastian Dipl.-Ing.,, 40221 Düsseldorf (DE); Beese, Jochen, 22848 Norderstedt (DE); Günther, Christopher Dipl.-Ing.,, 22085 Hamburg (DE)
(74) Representative: Eisenführ Speiser

(56) References cited:
- DE-A1- 2 753 388
- JP-A- 62 079 839
- US-A- 3 445 342
- US-A- 4 019 962
- US-A1- 2010 291 674
- US-A1- 2013 089 925

## Description

The application relates to a single-use bioreactor, suitable in particular for use in a bioreactor system, for growing biomass particles, comprising a head plate, a dimensionally stable container and a mixer, wherein the head plate and the container enclose a reaction chamber with the mixer positioned in the reaction chamber, the head plate having an inner side facing towards the reaction chamber, and an outer side which faces away from the reaction chamber and which has a plurality of connectors. The single-use bioreactor can be suitable for use in a parallel bioreactor system. Preferred application areas of the single-use bioreactor are microbiology and/or cell culture. A preferred application area can also be polymerase chain reaction (PCR), for example. The term biomass particles is intended to embrace any cells, including plant, animal bacteria, insect, fungus, yeast, or hybridoma cells, which can be grown in a culture medium.

Further, a baffle for use in such a single-use bioreactor is described.

Further, the invention relates to a process for manufacturing such a single-use bioreactor and a process for operating such a single-use bioreactor.

Bioreactors, which are also frequently referred to as fermenters, enclose a reaction chamber in which biological or biotechnological processes can be carried out on a laboratory scale. Such processes include, for example, the cultivation of cells, microorganisms or small plants under defined, preferably optimized, controlled and reproducible conditions. Bioreactors mostly have a plurality of connectors via which the primary and secondary substances, as well as various instruments, such as sensors, can be introduced to the reaction chamber or to which fluid conduits, for example, in particular gas conduits such as gassing conduits or exhaust gas conduits, can be connected. In addition, bioreactors generally include a mixer, the shaft of which can be made to rotate by a drive, as a result of which a stirring member connected torsionally rigidly to the mixer shaft is likewise made to rotate, thus mixing the substances present in the reaction chamber. It is also possible for two or more stirring members, mostly spaced axially apart, to be arranged on and connected to the mixer shaft. The stirring member or stirring members may also be integral with the mixer shaft.

Bioreactors are preferably used in bioreactor systems, preferably in parallel bioreactor systems, not only in the field of cell cultivation but also in microbiological applications. Parallel bioreactor systems are described in DE 10 2011 054 363.5 and DE 10 2011 054 365.1, for example. In such bioreactor systems, a plurality of bioreactors can be operated in parallel and controlled with higher precision. High-throughput experiments that are well reproducible and scalable can be carried out in the individual bioreactors, even with small operating volumes. The laboratory scale of the bioreactors to which the invention relates involves volumes of up to 10 I, or more specifically with a total reaction chamber volume of approximately 0.35, 1.5, 5, 10 I, with a working volume ranging between about 60 -250 ml, about 250-1300 ml, 1-3 I, 2.5-10 I. However, a general application range of bioreactors can be from about 20 ml working volume up to about 200 I of working volume, in some applications even up to 1000 or 3000 I.

In the cell culture field, such parallel bioreactor systems are used, for example, for test series for process optimization based on statistical planning methods (design of experiments DoE), for process engineering and in research and development, for example to cultivate different cell lines such as Chinese hamster ovary (CHO), hybridoma or NSO cell lines. In the context of the present application, the expression "cell culture" is specifically understood to mean the cultivation of animal, human or plant cells in a nutrient medium outside the organism.

In the field of microbiology, parallel bioreactor systems are likewise used for test series for process optimization based on statistical planning methods (design of experiments DoE), for process engineering and in research and development, for example to cultivate various microorganisms, in particular bacteria or fungi, such as yeast.

Due to limitations of space in most laboratories, minimal space requirements and in particular small footprints are striven for, not only for bioreactor systems but also for the bioreactors themselves.

Bioreactors used in laboratories are often made of glass and/or metal, in particular of stainless steel, as the bioreactors must be sterilized between different uses, preferably by steam sterilization in autoclaves. Sterilizing and cleaning reusable bioreactors is a complex process. The sterilization and cleaning process can be subject to validation, and needs to be precisely documented for each individual bioreactor. Residues in a bioreactor which has not been fully sterilized can falsify the results of a subsequent process, or render them useless, and may cause disruption of the subsequent process. Furthermore, the sterilization process may also expose individual components or materials in bioreactors to stress and strain, and in some cases can damage them.

Single-use bioreactors provide an alternative to reusable bioreactors and are used to carry out just one biological or biotechnological process before being disposed of. By providing a new single-use bioreactor for each process, and one that is preferably sterilized during the production process, it is possible to reduce the risk of (cross-) contamination, while simultaneously obviating the need to perform and document the impeccable cleaning and sterilization of a previously used bioreactor. Single-use bioreactors are often designed as flexible containers, for example as bags, or as containers having walls that are flexible in sections thereof at least. Single-use bioreactors with flexible containers are known for example from DE 10 2006 020 813 B3, DE 20 2007 005 868 U1, EP 2 231 316 B1 or JP 6279839 (A). However, one disadvantage of these single-use bioreactors with flexible walls, *inter alia,* is that they cannot be used in parallel bioreactor systems, which are designed for dimensionally stable, reusable bioreactors. A dimensionally stable bioreactor is understood to be rigid, i.e. to substantially retain its shape under intended operational conditions.

Further, for reasons of scalability of processes to different scale, qualitative comparable and particularly defined fluid dynamics (particularly during stirring) in the cultivation chamber are important. This requirement cannot be realized with bioreactors with flexible walls or can be realized only with extensive additional measures.

Dimensionally stable single-use bioreactors are known for example from US 2008/0131957 A1, US 2010/0291674 A1 or DE 10 2005 062 052 A1. However, these known, dimensionally stable single-use bioreactors are high-priced, on the one hand, and on the other hand are designed for pharmaceutical process engineering and pharmaceutical production processes. These are used for cell culture processes, in particular, and are therefore specifically designed and adapted for such cell culture processes. Different requirements must be met for applications in microbiology, however, not only with regard to the prices that can be charged on the market, but also, for example, with regard to appropriate design and to the materials that can be used. In particular, applications in microbiology are associated with challenging demands related to application of mechanical energy, heat transfer, and/or gas exchange rates, which can be higher by one or several magnitudes than in cell culture applications. Dimensionally stable single-use bioreactors known from the prior art are unsuitable, therefore, for use in microbiology research or in process engineering, for example.

A vessel for containing a culture growth medium which has an open mouth and a removable head plate with an impeller in the vessel for agitating the growth medium and a baffle assembly in the vessel for establishing a path through the medium for circulating a heat exchange medium to control the temperature of the growth medium is described in US 3,445,342.

It is therefore an object of the present invention, to provide a single-use bioreactor and a process for manufacturing and operating the same, which are particularly suitable for applications in microbiology. It is a further object of the invention, to provide for a single-use bioreactor and a process for manufacturing and operating the same, which feature enhanced operating characteristics for use a microbiology research or process engineering.

According to a first aspect of the invention, it is provided a single-use bioreactor according to claim 1.

The invention is based on the finding that specific advantages in applications of a single-use bioreactor in microbiology, in particular regarding fluid flow, mixing efficiency, and temperature control, can be achieved by providing at least one baffle within the reaction chamber which is projecting from the head plate and which has an inner fluid compartment. In the following, the inner fluid compartment of the baffle is sometimes in short referred to as fluid compartment. Equally, the single-use bioreactor is sometimes in short referred to as bioreactor.

The baffle is preferably used as a cooling baffle. The inner fluid compartment can be provided with a fluid flow, in particular a cooling fluid flow, such that the baffle at the same time functions as a baffle for improving flow and stirring characteristics within the reaction chamber and as a temperature control device in particular for cooling the contents within the container. External cooling baffles as disclosed in JP 6279839 (A) or EP 2 231 316 B1 are not located within the reaction chamber of the flexible single-use bioreactor but rather on a reusable outer frame or support. Therefore, it is possible to reuse the complex and thus expensive structure of external cooling baffles with multiple single-use flexible bioreactors. Baffles that are located within a single-use bioreactor as disclosed in DE 20 2007 005 868 U1, on the other hand, are simple and thus easy and cost effective to produce and do not comprise any additional functions such as cooling. In dimensionally stable single-use bioreactors as disclosed in US 2008/0131957 A1 or DE 10 2005 032 052 A1, baffles are formed within the outer wall of the container, which makes them easy and cost effective to manufacture and neither are located within the reaction chamber nor extending from the head plate. In contrast to such existing solutions, the invention is based on the finding that by providing at least one baffle extending from the head plate into the reaction chamber a cost effective construction has been found that allows for the added function of an inner fluid compartment of the at least one baffle in a dimensionally stable single-use bioreactor.

In addition or alternatively to providing the inner fluid compartment with a fluid flow, a tempered cooling finger, for example comprising or consisting of metal, preferably steel, or a cooling element, like a Peltier element, can be introduced into the inner fluid compartment.

The single-use bioreactor according to the invention has the advantage of providing for a cooling function together with enhanced fluid flow characteristics in a single element, namely the cooling baffle, and thus reducing the number of elements and reducing cost for providing those elements. In particular, by providing a baffle extending from the head plate, the costs can be kept in a region applicable for a single-use bioreactors.

The single-use bioreactor according to the invention includes two or more such baffles. Preferably, four such baffles can be present. It is further preferred, that such a plurality of baffles is constructed equally or at least similarly. In the following, when details are described with respect to one baffle, those details likewise apply to at least one baffle, two, three, four or more baffles, i.e. also to a plurality of baffles.

The advantages of the single-use bioreactor particularly apply for applications in microbiology with operating temperatures in a typical range of 20-40°C, in particular of about 37°C. Stirring speeds usually depend on the volume to be stirred. Maximum stirring speeds can be of about 2000 rpm for a volume of 0.3 I, 1600 rpm for a volume of 1 I, 1300 rpm for a volume of 3 I, 800-1000 rpm for a volume of 10 I, or less than 800 rpm for a volume of more than 40 I. Minimum stirring speeds can be of about 1/100 to 1/30 of the afore-mentioned maximum stirring speeds.

In practice, single-use bioreactors are mainly used for cell cultivation. In cell cultivation, usually stirred-tank reactors are used and operated at temperatures in a typical range of 20-40°C, in particular of about 37°C. Further, oxygen demand and thus oxygen transfer rate as well as temperature control are of lower importance since the usually are not limiting the cultivation process or can be handled with relative ease due to the low growth rate of the cells.

In microbiology applications, however, increased heat development due to high agitator speed and high biological activity of the cells of up to 80 W/I in high cell density fermentation has to be dealt with. Further, in stirred-tank reactors operated with high agitator speed a high application of energy is necessary to disintegrate and distribute air bubbles and to provide for an optimized homogenization.

The single-use bioreactor according to the invention is based on the finding that a dimensionally stable single-use bioreactor can be used successfully in microbiology applications, in particular with a working volume between 350-1400 ml, when the provision of baffles to create turbulence and thus prevent the forming of a vortex inside the reaction chamber is combined with an inner fluid compartment of the baffle for cooling the contents in the reaction chamber. The single-use bioreactor of the invention allows to produce the assembly with low enough production costs for sensibly priced single-use bioreactors by providing a baffle extending from the head plate into the reaction chamber with an inner fluid compartment in this baffle.

Preferably, the container and the head plate have circular cross sections and are connected to each other in a fluid tight manner, for example by welding or gluing. Welding or gluing is preferred over a mechanically sealed connection since a fluid tight connection can be created easier, cheaper and/or more reliable. Preferred welding techniques relate to plastic welding or heat sealing and include, for example, ultrasonic welding, laser welding, welding using infra-red radiation, or thermal welding. A welding technique has the advantage over gluing that no solvents are needed. Laser welding is particularly applicable if the head plate and/or the container is transparent for the laser. Welding using infra-red radiation has the advantage over ultrasonic welding that no or at least less dust and/or particles are formed. However, thermal welding or, in particular, welding using infra-red radiation, need enough space between welding seams to account for heat development.

The container preferably comprises a bottom and a sidewall, the bottom preferably having the shape of a circle and the sidewall preferably having the shape of a cylinder, and wherein preferably the container is integrally formed. Preferably, the head plate and/or the container comprise or consist of a material which conforms to United States Pharmacopeia (USP) Class VI requirements, preferably a plastic material, in particular polyamide, polycarbonate, polymethylpentene, polypropylene, or polystyrene. It is further preferred that the head plate can be manufactured in an injection molding process and is integrally formed. Further, it is preferred that also the container is integrally formed and can be manufactured in an injection or blow molding process. Preferably, also the head plate is dimensionally stable, i.e. substantially retains its shape under intended operational conditions.

The mixer preferably projects from the head plate into the reaction chamber and is preferably arranged at the center of the head plate or along a central axis of the bioreactor, respectively. The reaction chamber encloses a total volume of which a certain part is the working volume, i.e. the volume that is filled with the contents or substances used when operating the bioreactor. Generally, a minimum and a maximum working volume is indicated for a specific bioreactor of a specific size, and particular minimum and maximum fill levels are preferably indicated. In general, single-use bioreactors can have working volumes of about 20ml up to 2001, in some applications even up to 1000 or 3000 I. For the single-use bioreactor according to the present invention, volumes of up to 10 I are preferred, or more specifically a total reaction chamber volume of approximately 0.35, 1.5, 5, 10 I, with a working volume ranging between about 60 -250 ml, about 250-1300 ml, 1-3 I, 2.5-10 I. Preferably, if the single-use bioreactor is increased in its volume, the relative size of the baffle(s) is increased accordingly.

The inner fluid compartment of the baffle preferably is a hollow interior of the baffle, in particular of the whole baffle or a part of the baffle. The inner fluid compartment is adapted for a fluid flow therein or there through, respectively. Preferably, the baffle has at least one fluid connector as an inlet and/or outlet for fluid flow to and/or from the fluid compartment. The fluid flow within or through the inner fluid compartment is directed from an inlet to an outlet. Preferably, a fluid flows through a larger part of the inner fluid compartment on its route from the inlet to the outlet. To provide fluid to the at least one fluid connection, in particular to the inlet, preferably a fluid reservoir and a fluid pump are provided and connected to the at least one fluid connection. Further it is preferred, that also the outlet is connected to the same or another fluid reservoir to discharge fluid from the inner fluid compartment, by the same or a further fluid pump. In particular, a circular flow of fluid from the fluid reservoir to the inlet of the inner fluid compartment, through the inner fluid compartment, out of the inner fluid compartment, through the outlet and back to the fluid reservoir is preferred. Further, it is preferred that a temperature control unit is associated to the fluid reservoir to provide fluid to the inner fluid compartment with a specific temperature, in particular a temperature that is lower than the temperature within the reaction chamber during operation. The fluid used for flowing through the inner fluid compartment may be water. In order to reach very low temperatures of the fluid, an anti freezing agent might be added to the water or an anti freezing fluid may be used.

The baffle has first and second, opposite ends, wherein the first end is the end of the baffle adjacent the head plate and the second end is the end of the baffle extending to the into the reaction chamber. Preferably, the first end of the baffle is connected to the inner side of the head plate. Preferably, the first end of the baffle is connected to the inner side of the head plate in a fluid tight manner, preferably by welding or gluing. Welding or gluing is preferred over a mechanically sealed connection since a fluid tight connection can be created easier, cheaper and/or more reliable. Preferred welding techniques relate to plastic welding or heat sealing and include, for example, ultrasonic welding, laser welding, welding using infra-red radiation, or thermal welding.

The second end of the baffle preferably is located close to the bottom of the container. The inlet and the outlet are located at the first end of the baffle.

It is particularly preferred to pump fluid with a temperature below 15°C, in particular with a temperature of about 0-14°C, to the inner fluid compartment. In certain applications, a temperature of the cooling fluid below 0°C, in particular -5°C to 0°C can be preferred for a particular efficient heat transfer.

It is particularly preferred to pump a cooling fluid to a lower region of the inner compartment, preferably via an inlet tube. Preferably, the fluid flow centimes from the lower region of the inner compartment close to the second end of the baffle back to an outlet at the first end of the baffle with the fluid coming in contact with as much of an inner side of the outer wall of the baffle as possible to provide for effective cooling.

Preferably, the baffle extends from the head plate beyond a maximum fill level of the reaction chamber, preferably beyond a minimum fill level of the reaction chamber. By choosing a length of the baffle between its two ends that makes the baffle long enough to reach into the working volume of the reaction chamber, in particular into the minimum working volume of the reaction chamber, it can be ensured that both the cooling effect and the creation of turbulence is induced into the working volume. In particular, it is preferred that also the inner fluid compartment of the baffle extends beyond a maximum fill level of the reacting chamber, preferably beyond a minimum fill level of the reaction chamber.

An outer wall of the baffle preferably surrounds the fluid compartment and separates the fluid compartment from the reaction chamber, in particular in a fluid tight manner. Also, the second end of the baffle is a closed end, in particularly closed in a fluid tight manner. The first end of the baffle can be an open end which is connected to the head plate, in particular the inner side of the head plate, in a fluid tight manner. In particular, it is preferred that the fluid compartment of the baffle is fluid tight, i.e. leak proof, against the reaction chamber. In this way it can be prevented that a fluid circulating in the fluid compartment can leak into the reaction chamber and thus distorted the processes in the reaction chamber.

In particular, it is preferred that the fluid compartment of the baffle is adapted to withstand fluid pressure up to 50 kPa. Preferably, the fluid is pumped through the fluid compartment via a fluid pump with a certain pressure. Therefore, the fluid compartment is adapted to withstand such a fluid pressure from within. A maximum fluid pressure of 50 kPa (0,5 bar) is preferred so that the bioreactor is complying with low pressure regulations. While it can be preferred that the baffle is adapted to withstand fluid pressure of more than 50 kPa, it is preferred that the fluid provided to the baffle has a maximum fluid pressure of 50 kPa (0,5 bar) due to safety regulations.

In a further preferred embodiment of the invention, it is preferred that the at least one fluid connector is adapted to connect the inner fluid compartment of the baffle to the outer side of the head plate. A particular advantage can be realized when the fluid connection is accessible from the outer side of the head plate. In this way, the fluid compartment of the baffle can be connected to a fluid reservoir via the outer side of the head plate, where already a plurality of further connectors is present. In this way, the provision of fluid to the inner fluid compartment can be realized at the same place, namely the outer side of head plate, where other fluids and/or substances are provided to the reaction chamber. In particular, by avoiding a provision of fluid to the inner fluid compartment through the container of the bioreactor, the applicability of the single-use bioreactor in a parallel bioreactor system, where the containers of the bioreactors are placed in receptables, is ensured.

Regarding the fluid connection, it can be preferred that the inlet and the outlet are arranged coaxially within one fluid connector. In this embodiment, only one fluid connection per baffle is present and the inlet and outlet for supplying and discharging the fluid to and from the inner fluid compartment are arranged coaxially. Preferably, the inlet has a smaller diameter than the outlet and is arranged within the outlet.

Alternatively, it can be preferred that the baffle comprises two fluid connectors, one as an inlet and one as an outlet, wherein preferably the inlet and the outlet are spaced from each other. In this embodiment, a fluid connection each for the inlet and the outlet is provided.

According to a further preferred embodiment, the cross section of the baffle at its first end at the inner side of the head plate is larger than at its second end. The baffle, for example, can be tapered from its first end towards its second end. The baffle can be evenly tapered or the diameter can be reduced stepwise towards the second end.

In particular, it is preferred that the baffle, in particular the inner fluid compartment, has a substantially enlarged cross section in a connection region close to its first end to accommodate spaced apart inlet and outlet.

A larger cross section at the second end of the baffle has the advantage that the fluid flow from the inlet into the fluid compartment and from the fluid compartment through the outlet can be facilitated. In particular, if an embodiment with spaced apart inlet and outlet is used, it is preferred that the enlarged cross section extends from the first end of the baffle over a connection region adjacent this first end. This connection region preferably has an extension of less than a fourth, a fifth, a sixth or an eighth of the total length of the baffle. The cross section of the connection region preferably is larger than a remaining region of the baffle, in particular a remaining region of the inner fluid compartment, by a factor of 1.2, 1.5, 1.6, 1.8, 2, 2.2 or more. The afore-mentioned dimensions are particularly preferred for single-use bioreactors with working volumes between 350-1400 ml. In larger single-use bioreactors, in particular working volumes of 101 and larger, the relation of a cross sectional area at the first and second end of the baffle can change or even reverse.

In a further embodiment, preferably the at least one fluid connector comprises a connector on the outer side of the head plate adapted for connecting a tube thereto. In this embodiment, the fluid connection comprises a connector similar to the plurality of connectors already present on the outer side of the head plate for easy access and easy handling.

As already mentioned, the bioreactor can comprise more than one baffle. Preferably, the single-use bioreactor comprises a plurality of baffles, preferably four baffles, which are preferably arranged circumferentially equidistant. The plurality of baffles are preferably spaced apart form each other and located inside the reaction chamber close to the inner wall of the container and further preferably spaced equidistant along the inner perimeter of the container, while spaced from this inner perimeter.

Further, the plurality of baffles preferably is arranged to allow for fluid flow in series through the plurality of baffles, or in parallel through the plurality of baffles, or wherein two or more baffles are arranged to form a group in which the baffles are arranged for fluid flow in series through the baffles of the group, and wherein two or more of such groups are arranged for fluid flow in parallel through the two or more of such groups.

If two or more baffles are provided, they may be connected in series or in parallel or a combination thereof. When the baffles are connected to allow for fluid flow there through in parallel, the inlet and outlet of each of the baffles preferably is connected to a fluid reservoir. When two or more baffles are connected to allow for fluid flow there through in series, a preferable connection is as follows: The inlet of the first baffle is connected to a fluid reservoir, the outlet of the first baffle is connected to the inlet of the next baffle, the outlet of the next baffle is connected to the inlet of the last baffle and the outlet of the last baffle is connected to a further or the same fluid reservoir. In this way, fluid will flow through the first baffle, through one or more next or further baffles and through a last baffle before returning to the reservoir.

Groups of baffles can be formed wherein the baffles within a group are connected to allow for fluid flow in series there through while the different groups of baffles are arranged in parallel. It is particularly preferred to have two groups of two baffles each, i.e. two pairs of baffles.

Preferably, the single baffles of the two or more of such groups are positioned such that the baffles of different groups are arranged circumferentially alternating. In the case that two groups with two baffles each are provided, i.e. two pairs of baffles, those two pairs are preferably arranged crosswise.

In a particularly preferred embodiment, all baffles are arranged for fluid flow in parallel there through to increase the cooling effect.

In a particularly preferred embodiment, the baffle is arranged such that a space exists between the baffle and an inner wall of the container, wherein this space preferably is equal to or lager than 1/100 of the inner diameter of the container, in particular equal to or lager than 1/50 of the inner diameter of the container. Providing for baffles within the reaction chamber and with a distance to the inner wall of the container and further preferably with a distance to the bottom of the container has the advantage that dead spaces can be avoided or at least reduced. Dead spaces are prone to the forming of unwanted cell agglomerates due to less exposure to stirring agitation and turbulence. By providing a space between the inner surface of the container and the baffles, it is ensured that contents within the reaction chamber can freely flow around the baffles.

Preferably, a radial extension of the baffle is equal to or lager than 1/6 of the inner diameter of the container, in particular equal to or lager than 1/12 of the inner diameter of the container. A radial extension of the baffle is the extension of the baffle in a radial direction of a bioreactor with a cylindrical container. A radial extension of the baffle equal to or larger than 1/6, in particular equal to larger than 1/12 of the inner diameter of the container has proven to be particularly advantageous to create desired turbulences within the reaction chamber. A particularly preferred radial extension of the baffle is equal to or lager than 1/10 of the inner diameter of the container.

A preferred thickness of the baffle orthogonal to its radial extension and orthogonal to its extension between its first and second ends is between 0,2 and 0,8 of its radial extension, in particular between 0,4 and 0,6 of its radial extension.

It is further preferred that a second end of the baffle has a shape that is adapted to the shape of the container, in particular the shape of a curved connection of the bottom to the walls of the container. Particularly, a radially outer corner of the second end of the baffle is rounded or slanted.

In a further preferred embodiment a spacer is positioned between the baffle and the inner wall of the container, preferably in a lower third, in particular a lower fourth or fifth of the baffle. A spacer can be provided to ensure a distance between the inner wall of the container and the baffle during operation of the bioreactor, in particular when high agitator speeds are used.

The indication of a lower part of the baffle refers to a region towards the second end of the baffle and/or towards the bottom of the container. For example, the spacer can be a ring surrounding the perimeter of the baffle, a projection on the baffle and/or the inner wall of the container, a ring adjacent the inner wall of the container or projecting from the inner wall of the container, or a ring surrounding a plurality of baffles on their radially outward sides. Further, the spacer can be a ring or frame connecting a plurality of baffles on their radially inward sides. In this case, the spacer preferably has dimensions that ensure that the baffles are connected to each other such that radially opposite baffles are spaced apart from each other such that the distance between their radially outward sides is smaller than an inner diameter of the container.

Further, two or more spacers can be provided preferably at different heights of the baffle or the container, respectively. Preferably, each of a plurality of baffles is spaced from the inner wall of the container by an individual spacer. Alternatively, two or more baffles can be spaced from the inner wall of the container by one or more shared or common spacers.

Further, it is preferred that the baffle, in particular an outer wall of the baffle, comprises or consists of a material which conforms to United States Pharmacopeia (USP) Class VI requirements, preferably a plastic material, for example polyamide, polycarbonate, polymethylpenten, polypropylene or polystyrene.

It is particularly preferred that an outer wall of the baffle, particularly a section of the outer wall of the baffle extending beyond a maximum fill level of the reaction chamber, comprises a thermal conducting additive, or preferably consists of a composite material comprising a plastic matrix and a thermal conducting material, or includes such a composite material. Preferably, also the thermal conducting additive or the composite material conforms to United States Pharmacopeia (USP) Class VI requirements.

This embodiment has the advantage that the thermal conductivity of the outer wall of the baffle can be enhanced. A thermal conducting additive or a thermal conducting material is meant to be a material with a high thermal conductivity, in particular a thermal conductivity that is higher than the remaining material of the baffle, in particular a thermal conductivity of more than 1 λ (1 W/m•K) preferably more than 2, 5, 10, 25, or 50 λ. Such an increased thermal conductivity of the baffle, in particular in the region between the maximum fill level and the second end of the baffle, improves heat transfer between a cooling fluid in the fluid compartment of the baffle and the contents in the reaction chamber.

Preferably, an outer wall of the baffle, particularly a section of the outer wall of the baffle extending beyond a maximum fill level of the reaction chamber has a thickness of less than 2,5 mm or 1/50 of the inner diameter of the container. It is particularly preferred that the thickness of an outer wall of the baffle, particularly the section between the maximum fill level and the second end of the baffle, is equal to or less than 2 mm, 1,5 mm, 1 mm, or 1/100 of the inner diameter of the container.

It is particularly preferred that the baffle is integrally formed, preferably in an injection or blow molding process.

In a preferred embodiment, an inlet tube is positioned within the fluid compartment extending from the inlet toward the second end of the baffle. This tube can be connected to a respective connector at the inner side of the head plate or can be formed integral with the head plate. Preferably, the tube extends from the inlet beyond the maximum fill level, preferably beyond the minimum fill level of the reaction chamber. Such a tube has the advantage that fluid provided to the inner fluid compartment of the baffle is routed within the tube towards a lower part of the baffle into a region close to the second end of the baffle or at least into a region within the working volume of the reaction chamber. In this way, fluid flow throughout the entire length of the baffle or at least through a larger part of the length of the baffle can be enhanced.

In a further preferred embodiment the fluid compartment comprises or consists of a conduit between the inlet and the outlet, wherein the conduit preferably has the shape of a labyrinth, a meander, a double helix or other form substantially extending over the whole baffle. Further, it is preferred that the conduit is adapted to withstand a fluid pressure of max 50kPa, wherein the conduit is adapted to allow for fluid to pass through the conduit with a maximum pressure of 50 kPa.

Such by conduit can enhance fluid flow through a larger part of the baffle, in particular close to the second end of the baffle. However, the conduit, in particular its form, is preferably constructed such that sufficient fluid flow can be achieved with a fluid pressure of a maximum of 50 kPa.

Depending on baffle geometry and fluid pressure thresholds, different flow velocities of the fluid in the inner fluid compartment will be realized.

In a further preferred embodiment, the baffle comprises at least one mounting element for mounting a spacer and/or a fluid connecting element, preferably a spacer ring and/or a fluid connecting ring, thereto. An element, like a spacer and/or a fluid connecting element, can preferably be connected to the baffle via at least one mounting element. A mounting element on the baffle preferably interacts with a corresponding mounting element on the spacer and/or a fluid connecting element to form a releasable or non-releasable connection. If a spacer element is mounted to the baffle, the connection can be releasable and for example be a snap-on connection. Alternatively, a spacer element can be connected to the baffle via welding or gluing or can be formed integral with the baffle. Preferred welding techniques relate to plastic welding or heat sealing and include, for example, ultrasonic welding, laser welding, welding using infra-red radiation, or thermal welding.

In case a fluid connecting element is mounted on the baffle, a fluid tight connection between the fluid connecting element and the baffle is preferred. The fluid connecting element can be used to connect the inner fluid compartments of two or more baffles together to allow for fluid flow in between, in particular if the baffles are connected to each other to allow for fluid flow in series.

In a further preferred embodiment, the inlet and/or outlet of the baffle are connected to a fluid reservoir and/or the outlet and/or inlet of a further baffle via tubing on the outer side of the head plate, via tubing on the inner side of the head plate, via a connecting ring positioned adjacent the inner side of the head plate or below a maximum fill level in the reaction chamber, or via tubing and/or a connecting ring formed within the head plate.

For parallel fluid flow through two or more baffles, preferably the inlet and outlet of the baffles are each connected to a fluid reservoir. However, if two or more baffles are connected to allow for fluid flow in series, a connection between those two or more baffles needs to be provided. Therefore, the outlet of one baffle can be connected to the inlet of a further baffle and so on, with the inlet of the first baffle being connected to a fluid reservoir and the outlet of the last baffle also being connected to a further or the same fluid reservoir. The inlets and outlets inbetween can be connected for example via tubing on the outer side of the head plate. Tubes of such tubing are preferably connected to respective connectors on the outer side of the head plate related to the inlet ore outlet, respectively. Alternatively, a connecting ring can be positioned within the reaction chamber to provide a fluid connection between the baffles. This connecting ring can access the inner fluid compartment of the baffles via an additional fluid connection. The connecting ring can be oval, circular, elliptical, angular, or of any other form suitable for creating a connection for fluid flow between the baffles.

It is also possible that only the inlet of a first baffle and the outlet of a last baffle are formed as fluid connections on the head plate and the outlet of the first baffle, the inlet of the last baffle as well as the inlet and outlet of baffles inbetween the first and second baffle are located on the part of the baffle positioned within the reaction chamber.

If a fluid connection between baffles is created within the reaction chamber, it is important that a fluid tight enclosure of the baffles and the connecting ring against the reaction chamber be provided. In particular, the complete fluid circulation space through one or more baffles and their respective connections to each other within the reaction chamber is preferably constructed in a fluid tight manner. In particular, the connecting ring can be connected to the baffles via welding or gluing. Further, the connecting ring and the baffles can be formed integral within each other. Preferred welding techniques relate to plastic welding or heat sealing and include, for example, ultrasonic welding, laser welding, welding using infra-red radiation, or thermal welding.

According to a further possibility, the connecting ring can be formed within the head plate and preferably is formed integral with the head plate. This is a particular advantageous embodiment to provide for a fluid tight connecting ring.

In a further preferred embodiment, the cross section of the baffle has substantially the form of a rectangle, a trapeze, a parallelogram, a circle, an ellipse, a racetrack, or a triangle, or a combination of such shapes.

Preferably, the baffle comprises, in particular a section of the baffle extending beyond a maximum fill level of the reaction chamber, a projecting edge.

The projecting edge preferably projects radially inwards, i.e. towards the center of the container, from the baffle. The projecting edge can be an elongation of one of the sidewalls of the baffle in a radially inward direction. The edge extends preferably at least between the maximum fill level and the second end of the baffle. Such a projecting edge has the advantage of providing a flow breakaway edge to introduce turbulence into the fluid flow within the reaction chamber. To increase the turbulence enhancing characteristics of the projecting edge, the edge projects from the remaining cross section of the baffle preferably by at least 0,5 mm, preferably at least 1 or 2 mm or at least 1/10 of the radial extension of the baffle. The projecting edge preferably has a thickness equal to or less than its radial extension form the remaining cross section of the baffle. It is particularly preferred that the projecting edge is chamfered. Further, it is preferred that the inner fluid compartment of the baffle does not extend to the projecting edge, i.e. there is no fluid flow through the projecting edge.

One advantage of the projecting edge is that the slim form of this edge enhances the development of desired turbulences. As opposed to the rest of the baffle, which has a larger cross section compared to the projecting edge, by providing for a baffle with a main cross section large enough for having an inner fluid compartment therein for cooling plus providing for a projecting edge to enhance the development of turbulences, an advantages construction is achieved.

Preferably, the baffle is arranged such that the stirred medium impacts the baffle substantially orthogonal on a sidewall of the baffle having the projecting edge.

Further, a baffle for use in a previously described single-use bioreactor is described, wherein the baffle has an inner fluid compartment.

According to another aspect of the invention, it is provided a biotechnological device comprising a previously described single-use bioreactor, further comprising a fluid reservoir, a fluid pump and a temperature control unit.

As to the advantages, preferred embodiments and details of the baffle and the biotechnological device, reference is made to the corresponding aspects and embodiments of the single-use bioreactor described above.

According to a further aspect of the invention it is provided a process for manufacturing a single-use bioreactor according to claim 12.

According to yet a further aspect of the invention, it is provided a process for operating at least one previously described single-use bioreactor, comprising the steps: providing at least one previously described single-use bioreactor, providing a fluid reservoir, a fluid pump and a temperature control unit, connecting the fluid reservoir to the inner fluid compartment of the baffle, supplying fluid from the fluid reservoir to the inner fluid compartment of the baffle.

Finally, according to a further aspect the invention is directed to the use of a previously described baffle in a previously described single-use bioreactor.

As to the advantages, preferred embodiments and details of the process for manufacturing a single-use bioreactor, of the process for operating at least one single-use bioreactor, and of the use of a baffle, reference is made to the corresponding aspects and embodiments described above.

Preferred embodiments of the invention shall now be described with reference to the attached drawings, in which
- Fig. 1:: shows a longitudinal section of an example embodiment of a single-use bioreactor according to the invention;
- Fig. 2:: shows an enlarged detail of the upper left corner of Fig. 1;
- Fig. 3:: shows an enlarged detail of the lower left corner of Fig. 1;
- Fig. 4:: shows a cross section through the single-use bioreactor according to Fig. 1;
- Fig. 5:: shows a three dimensional view of a baffle used in the single-use bioreactor according to Fig. 1;
- Fig. 6:: shows a longitudinal section of the baffle according to Fig. 5;
- Fig. 7:: shows the head plate and baffles of the single-use bioreactor according to Fig. 1 with a first example of tubing connections,
- Fig. 8:: shows the head plate and baffles of the single-use bioreactor according to Fig. 1 with a second example of tubing connections; and
- Fig. 9:: shows the head plate and baffles of the single-use bioreactor according to Fig. 1 with a third example of tubing connections.

In the different figures, corresponding elements and/or elements with substantially the same function will be indicated with the same reference numerals.

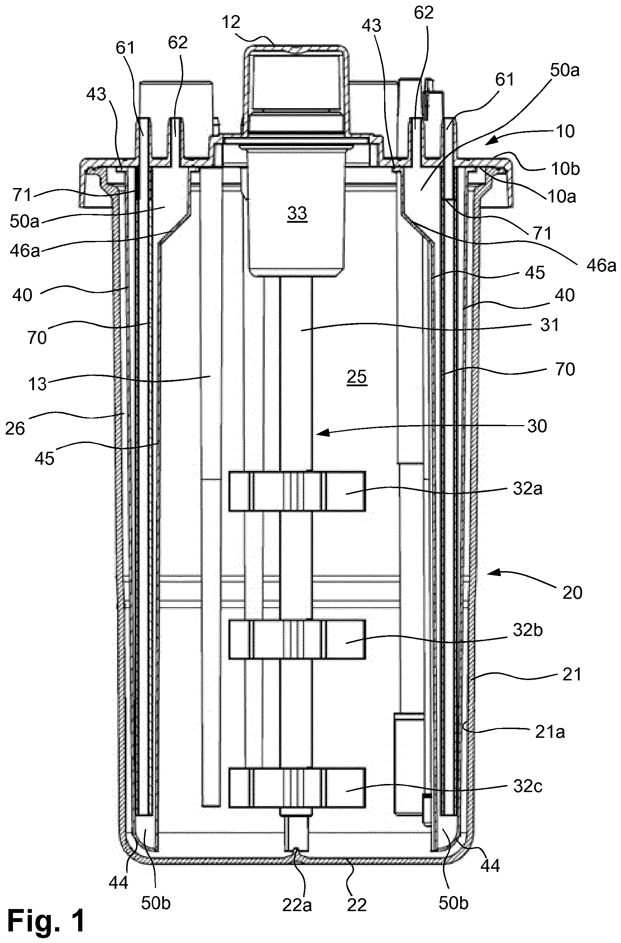

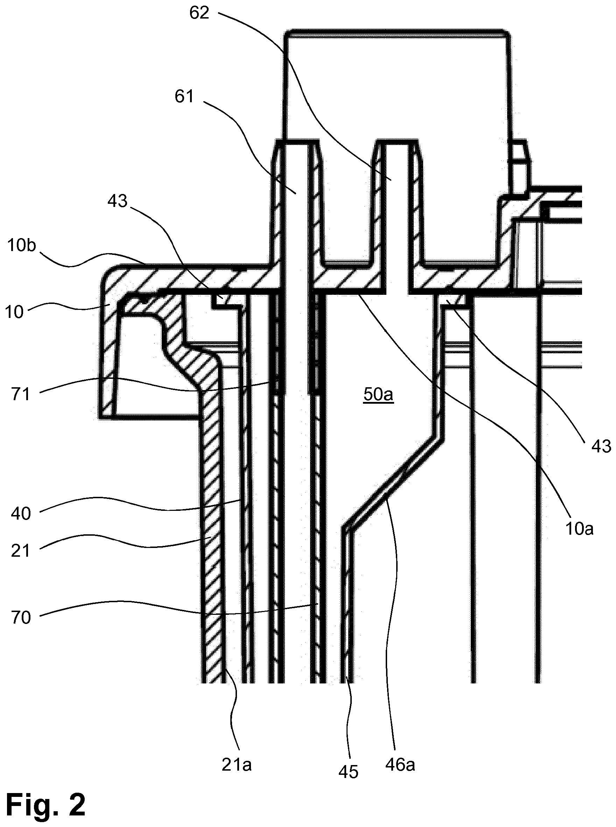

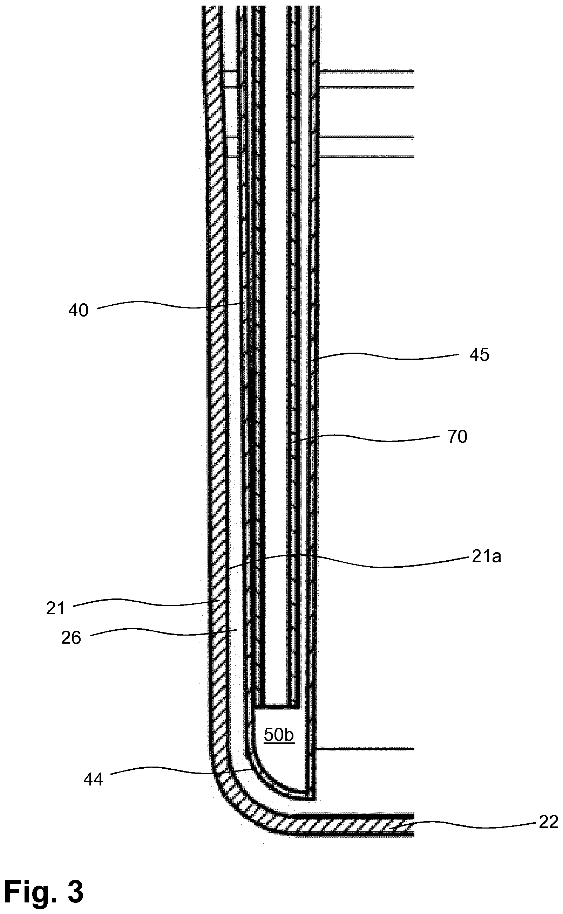

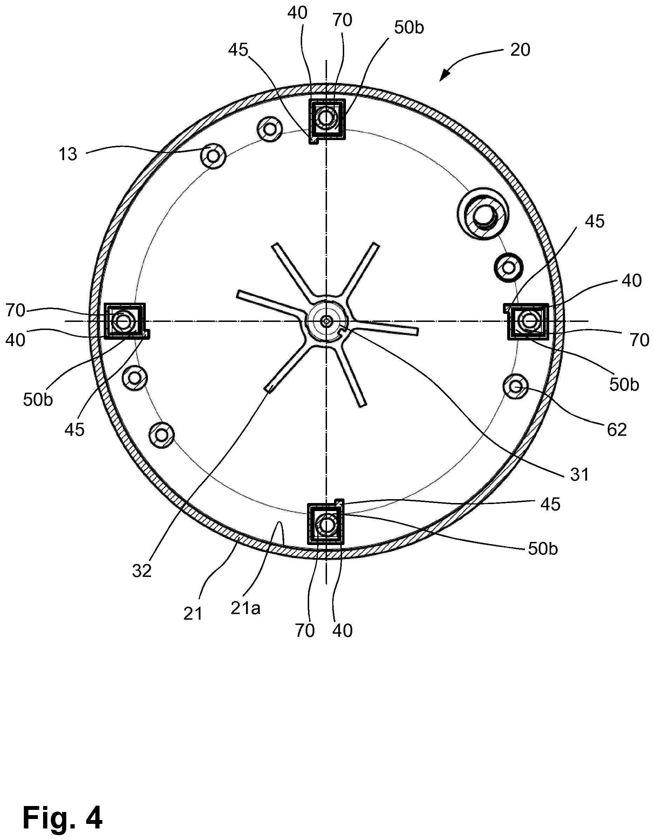

The example embodiment of a single-use bioreactor 1 according to the invention shown in figures 1-4 comprises a head plate 10, a dimensionally stable container 20 and a mixer 30. The head plate 10 and the container 20 enclose a reaction chamber 25. The mixer 30 is positioned within this reaction chamber 25. Head plate 10 has an inner side 10a facing towards the reaction chamber 25, and an outer side 10b which faces away from the reaction chamber 25 and which has a plurality of connectors 11. Head plate 10 is of integral construction and preferably comprises or consists of a material which conforms to United States Pharmacopeia (USP) Class VI requirements, preferably a plastic material, in particular polyamide, polycarbonate, polymethylpentene, polypropylene, or polystyrene and is preferably produced in an injection molding process, including the connectors 11 arranged on its outer side 10b and a plurality of dip tubes 13 arranged on its inner side 10a. Dip tubes 13 match a part of connectors 11, such that instruments, sensors, conduits or tubes can be inserted into and removed from the reaction chamber 25 through corresponding connectors 11 and through dip tubes 13. Connectors 11 are used to provide the substances necessary for the reaction process and/or to remove substances, such as gases produce during operation, from reaction chamber 25. Connectors 11 can also be referred to as overlay and dip tubes 13 as submerse or submersibles.

Tubes and connection materials which are used with the single-use bioreactor 1 and which can come into contact with reaction media are preferably made of materials with United States Pharmacopeia (USP) Class VI certification, such as polystyrene, polycarbonate, polyamide or silicone. The tubes to be used are preferably flexible tubes made of thermal plastic elastomers. Alternatively, rigid tubes, for example comprising or consisting of polystyrene, polycarbonate, or polyamide, can be preferred.

Head plate 10 further has a convex or outer projection portion 12 for accommodating bearing 33 of mixer 30. Mixer 30 has a mixer shaft 31 with an axis of rotation and three stirring members 32a, b, c. Stirring members 32a, b, c are Rushton type impellers with six blades. The three stirring members 32a, b, c are arranged equidistant of each other in a lower region of mixer shaft 31 such that preferably the lowest stirring member 32c is located below a minimum fill level and the upper most stirring member 32a is positioned below a maximum fill level.

Container 20 has a circular cross section with a circular bottom 22 and a cylindrical sidewall 21 with inner surface or inner sidewall 21a. Container 20 is of integral construction and bottom 22 and sidewall 21 are connected via a curved region. Head plate 10 and container 20 are connected to each other preferably by welding or gluing. In the center of bottom 22 of container 20, a projection 22a is positioned to engage the lower tip of mixer shaft 31 to provide stability for the mixer shaft 31 during rotation or agitation.

Stirring members 32a, b, c are attached torsionally rigidly to mixer shaft 31, with the result that rotation or agitation of mixer shaft 31 causes stirring members 32a, b, c to rotate as well. Mixer shaft 31 is mounted rotatable about a rotational axis in a bearing 33. Mixer 30 including bearing 33 is completely arranged in reaction chamber 25. Preferably, the upper end of mixer shaft 31 has a magnetic portion which can be coupled magnetically in an axial direction to a rotary drive (not shown). Such rotary drive can be substantially cylindrical with a cross sectional area which is substantially identical to the cross sectional area of the convex or centrally projecting portion 12 on head plate 10.

Single-use bioreactor 1 comprises four baffles 40 extending from the head plate 10 into the reaction chamber 25. Baffles 40 have an inner fluid compartment 50 and at least one fluid access. In the example depicted, baffles 40 have a first end 41, a second end 42 and a region 46 with an enlarged cross section closed to first end 41. Accordingly, inner fluid compartment 50 has a section 50a with an enlarged cross section and a section 50b with a smaller cross section. The sidewall of baffle 40has a slanted section 46a for reducing the enlarged cross section of region 46.

An inner diameter of the container 25 preferably is 105,3 mm, where a radial extension of the baffle 40 preferably is 1/10-1/12 of the inner diameter of the container. A space 26 between the baffle 40 and the inner surface 21a of the container 20 preferably is 1/50 of the inner diameter of the container 25.

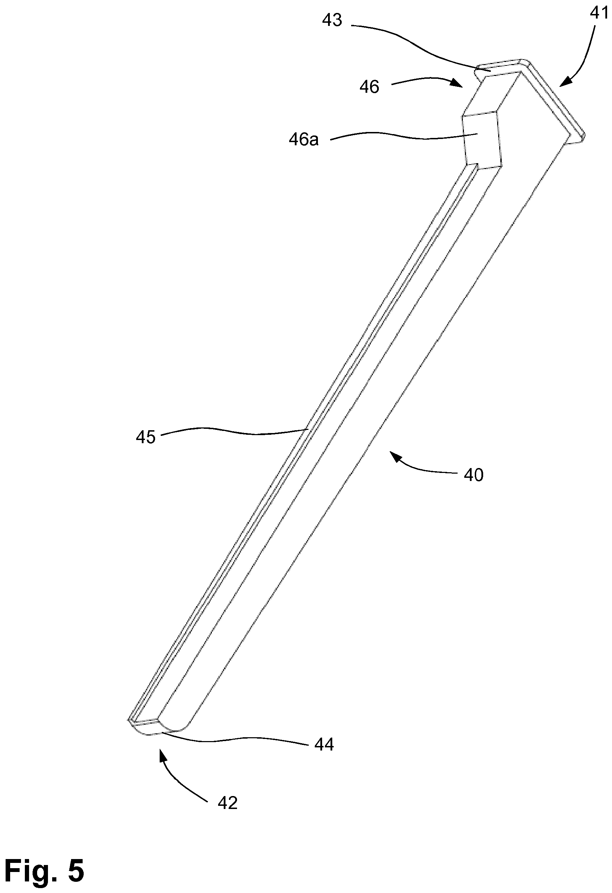

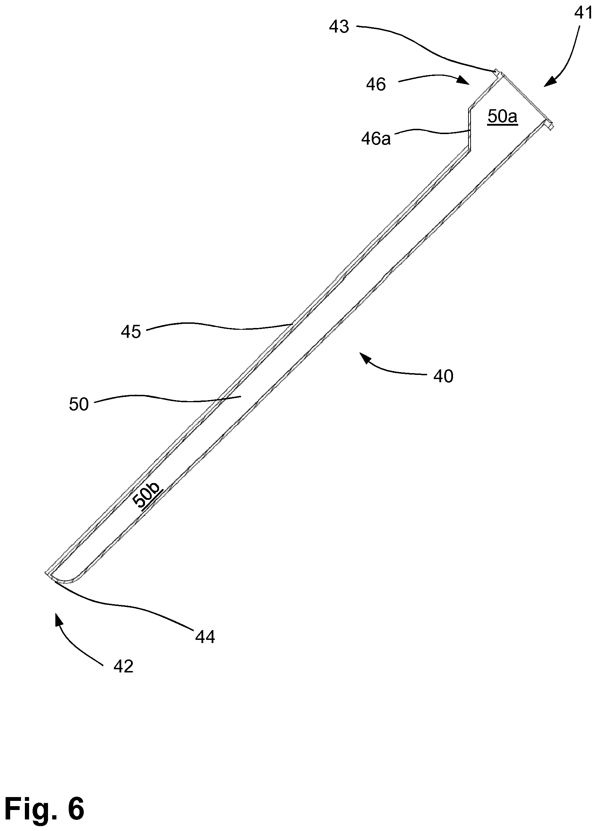

At the first end 41, baffle 40 has a flange 43 for connecting baffle 40 to the inner side 10a of head plate 10. The second end 42 of baffle 40 has a rounded outer corner 44, which matches the rounded connection between bottom 22 and sidewall 21 of container 20. The inner fluid compartment 50 of baffle 40 substantially extends throughout the whole baffle 40 and is surrounded by sidewalls of the baffle. In the example depicted, first end 41 of baffle 40 in the situation shown in figures 5 and 6 is an open end. However, since baffle 40 is connected to the inner side 10a of head plate in a fluid tight manner, inner compartment 50 of baffle 40 as shown in figures 1-3 is completely separated from the reaction chamber 25 in a fluid tight manner. Preferably, flange 43 at first end 41 of baffle 40 is connected to the inner side 10a of head plate 10 by welding or gluing.

On the outer side 10b of head plate 10 connectors 61 and 62 are present and function as fluid access as inlets and/or outlets for fluid flow to and from the fluid compartment 50. Fluid connector 61 is an inlet to fluid compartment 50, and particular the enlarged section of 50a of fluid compartment 50. On the inner side 10a of head plate 10, corresponding to inlet 61 is a further connector 71 for connecting an inlet tube 70 thereto. Alternatively, inlet tube 70 can be formed integral with connector 71 and head plate 10. Inlet tube 70 guides fluid supplied to inner compartment 50 via inlet 61 down to the lower end of baffle 40 such that the fluid coming from inlet 61 needs to travel down all the length of baffle 40 and from there flows on the outer side of inlet tube 70 back up to second end 41 of baffle 40, in particular back to enlarged section 50a of fluid compartment 50. Enlarged portion 50a of fluid compartment 50 provides access to fluid connector 62 functioning as a fluid outlet of the baffle.

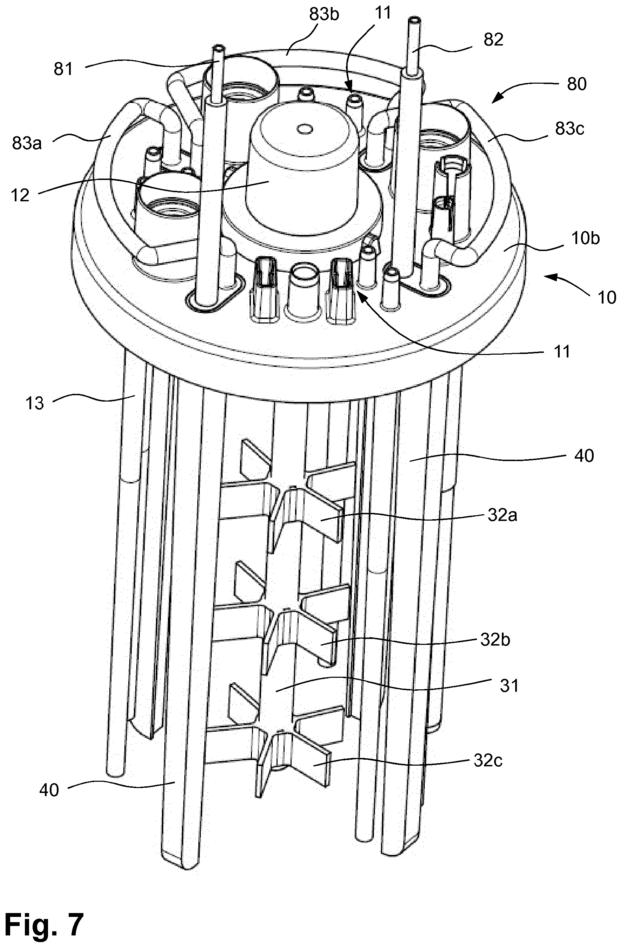

Different possibilities to connect fluid connectors 61 and 62 of baffles 40 to each other and/or a fluid reservoir (not shown) are depicted in figures 7-9. In figure 7, a tubing connection 80 for connecting the four baffles 40 in series is depicted. Tubing connection 80 is adapted to allow for fluid flow in series through the four baffles 40. The inlet of a first baffle is connected to a fluid reservoir (not shown) via a first tube 81. The outlet of this first baffle is connected via tube 83a to the inlet of a second baffle. The outlet of this second baffle is connected to the inlet of the third baffle via tube 83b. The outlet of the third baffle is connected to the inlet of fourth baffle via tube 83c. The outlet of the fourth and last baffle is connected via tube 82 to a further or the same fluid reservoir. Preferably, tube 81 for providing fluid to the baffles and tube 82 for discharging fluid from the baffles are connected to the same fluid reservoir to form a fluid circle. The fluid circle preferably comprises a fluid pump for circulation of fluid through this fluid system. Further, a temperature control unit is preferably associated with the fluid system for cooling the fluid.

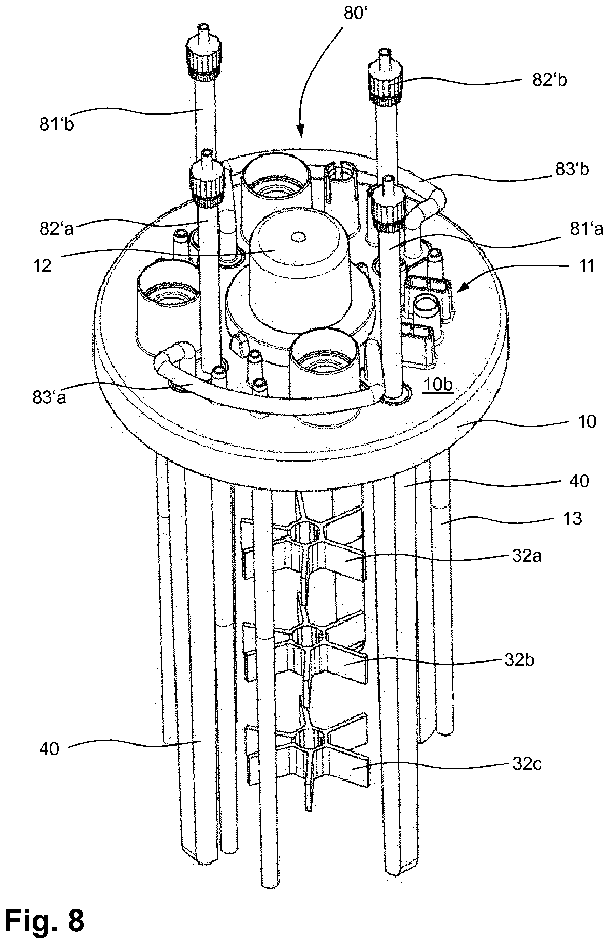

In the example depicted in figure 8, two pairs of baffles are arranged for fluid flow in parallel while the two baffles of each pair are arranged for fluid flow in series thereto. A first pair of baffles, in figure 8 the two baffles 40 in the front, are connected in series via tube 83'a, which connects the outlet of the first baffle two the inlet of the second baffle. The inlet of the first baffle is connected via tube 81'a to a fluid reservoir. Further, the outlet of the second baffle is connected via tube 82'a to the fluid reservoir. A second pair of baffles, in figure 8 the two baffles in the back, are also connected for flow in series via tube 83'b, which connects the outlet of a third baffle to the inlet of a fourth baffle. The inlet of the third baffle is connected via tube 81'b to the fluid reservoir and the outlet of the fourth baffle is connected to the fluid reservoir via tube 82'b. This arrangement of tubing connection 80' provides for fluid flow in series between the first and the second baffle on the one hand and the third and the fourth baffle on the other hand and further for fluid flow in parallel between those two pair of baffles.

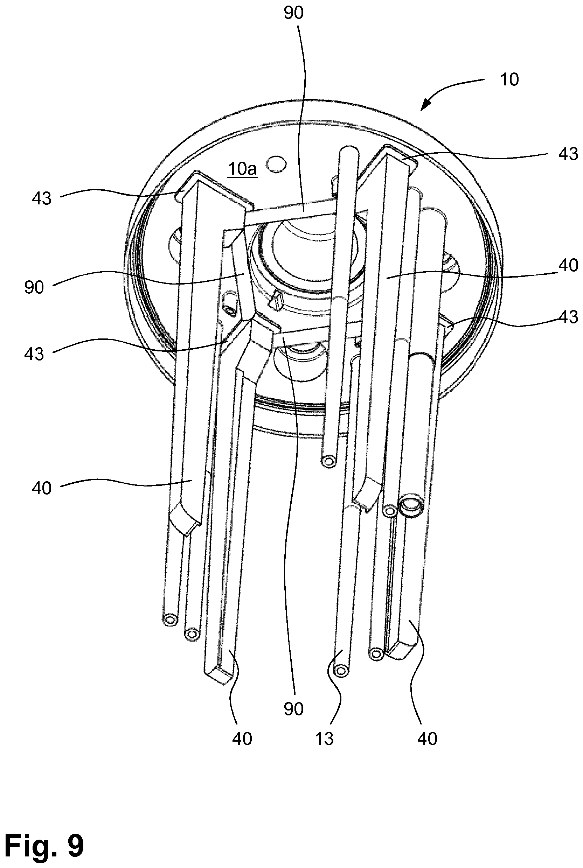

In the examples shown in figure 7 and figure 8, tubing connections 80, 80' are provided on the outer side 10b of head plate 10 and comprise several tubes. An alternative arrangement is depicted in the example of figure 9.

In figure 9, a connection between the baffles is provided on the inner side 10a of head plate 10. This tubing connection 90 has the form of an annular connecting ring and can provided separate from the head plate 10 or be made integral with the head plate 10. If tubing connection 90 is provided separate from the head plate, it is preferably located close to or adjacent the inner side 10a of head plate 10. However, it is also possible to provide fluid connecting ring 90 in a lower region of the baffles 40. If a fluid connection between the baffles 40 as shown in figure 9 is provided within the reaction chamber, it is preferred that this connection is fluid tight, for example by integrally forming, welding or gluing. The example depicted in figure 9 would also allow for fluid flow in series through the four baffles 40.

The fluid flow through the baffles 40 allows for effective cooling of the contents in the reaction chamber 25. Mostly, cooling of those contents will be desired. However, the fluid flow through the baffles 40 also allows for temperature control in general.

To further improve fluid flow characteristics within the reaction chamber, baffles 40 are provided with a projecting edge 45. This projecting edge 45 extends from the end of the enlarged region 46 to the second end 42 of the baffle. This projecting edge 45 projects from the remaining cross section of the baffle 40 and functions as a flow breakaway edge to increase the forming of turbulences within the reaction chamber 25. The projecting edges 45 are formed as elongations of one of the sidewalls of each of the baffles 40 in a radially inward direction, and the baffles 40 are arranged such that the stirred medium impacts the baffles substantially orthogonal on the sidewalls having the projecting edges 45. In this way, disadvantages resulting from the form of the baffles 40 due to the necessary inner fluid compartment 50 for the circulation of cooling fluid can be reduced significantly.

### REFERENCE SIGNS

- 1: single-use bioreactor
- 10: head plate
- 10a: inner side
- 10b: outer side
- 11: connectors
- 12: outer projection portion
- 13: dip tubes
- 20: container
- 21a: inner surface
- 21: sidewall
- 22a: projection
- 22: bottom
- 25: reaction chamber
- 26: space
- 30: mixer
- 31: mixer shaft
- 32a, 32b, 32c: stirring member
- 33: bearing
- 40: baffle
- 41: first end
- 42: second end
- 43: flange
- 44: outer corner
- 45: projecting edge
- 46: region with enlarged cross section
- 46a: slanted section
- 50: inner fluid compartment
- 50a: section with a enlarged cross section
- 50b: section with a small cross section
- 61: inlet
- 62: outlet
- 70: inlet tube
- 71: connector
- 80, 80': tubing connection
- 81, 82: tube
- 81'a, 81'b, 82'a, 82'b: tube
- 83a, 83b, 83c: tube
- 83'a, 83'b: tube
- 90: connecting ring

## Claims

1. A single-use bioreactor (1), suitable in particular for use in a bioreactor system, for growing biomass particles,
comprising a head plate, a dimensionally stable container (20) and a mixer (30), wherein the head plate (10) and the container (20) enclose a reaction chamber (25) with the mixer (30) positioned in the reaction chamber (25),
the head plate (10) having an inner side (10a) facing towards the reaction chamber (25), and an outer side (10b) which faces away from the reaction chamber (25) and which has a plurality of connectors (11), and
comprising two or more baffles, each baffle (40) extending from the head plate (10) into the reaction chamber (25), and each baffle (40) having an inner fluid compartment (50),
wherein each baffle has first and second, opposite ends, wherein the first end is adjacent the head plate and the second end is extending into the reaction chamber,
wherein each baffle has an inlet and an outlet adapted to direct fluid flow within or through the inner fluid compartment from the inlet to the outlet,
wherein for each baffle, the inlet and the outlet are located at the first end of the baffle.

2. The single-use bioreactor (1) according to at least one of the preceding claims, wherein the cross section of the baffle (40) at its first end (41) at the inner side (10a) of the head plate (10) is larger than at its second end (42).

3. The single-use bioreactor (1) according to at least one of the preceding claims, comprising a plurality of baffles (40), preferably four baffles (40), which are preferably arranged circumferentially equidistant.

4. The single-use bioreactor (1) according to at least one of the preceding claims, wherein the plurality of baffles (40) preferably is arranged to allow for fluid flow
- in series through the plurality of baffles (40), or
- in parallel through the plurality of baffles (40), or
wherein two or more baffles (40) are arranged to form a group in which the baffles (40) are arranged for fluid flow in series through the baffles (40) of the group, and wherein two or more of such groups are arranged for fluid flow in parallel through the two or more of such groups.

5. The single-use bioreactor (1) according to at least one of the preceding claims, wherein the baffle (40) is arranged such that a space (26) exists between the baffle (40) and an inner wall of the container (20), wherein this space (26) preferably is equal to or lager than 1/100 of the inner diameter of the container (20), in particular equal to or lager than 1/50 of the inner diameter of the container (20).

6. The single-use bioreactor (1) according to at least one of the preceding claims, wherein an outer wall of the baffle (40), particularly a section of the outer wall of the baffle (40) extending beyond a maximum fill level of the reaction chamber (25) comprises a thermal conducting additive, or preferably consists of a composite material comprising a plastic matrix and a thermal conducting material, or includes such a composite material.

7. The single-use bioreactor (1) according to at least one of the preceding claims, wherein an inlet tube (70) is positioned within the fluid compartment extending from an inlet toward a second end (42) of the baffle (40).

8. The single-use bioreactor (1) according to at least one of the preceding claims, wherein the baffle (40) comprises at least one mounting element for mounting a spacer and/or a fluid connecting element, preferably a spacer ring and/or a fluid connecting ring, thereto.

9. The single-use bioreactor (1) according to at least one of the preceding claims, wherein the inlet (61) and/or outlet (62) of the baffle (40) are connected to a fluid reservoir and/or the outlet (62) and/or inlet (61) of a further baffle (40) via tubing on the outer side (10b) of the head plate (10), via tubing on the inner side (10a) of the head plate (10), via a connecting ring positioned adjacent the inner side (10a) of the head plate (10) or below a maximum fill level in the reaction chamber (25), or via tubing and/or a connecting ring formed within the head plate (10).

10. The single-use bioreactor (1) according to at least one of the preceding claims, wherein the baffle (40) comprises, in particular a section of the baffle (40) extending beyond a maximum fill level of the reaction chamber (25), a projecting edge.

11. A biotechnological device comprising a single-use bioreactor (1) according to at least one of the preceding claims 1-10, further comprising a fluid reservoir, a fluid pump and a temperature control unit.

12. A process for manufacturing a single-use bioreactor (1), suitable in particular for use in a bioreactor system, for growing biomass particles, preferably a single-use bioreactor (1) according to at least one of the preceding claims 1-10,
comprising the steps:
- providing a head plate (10), the head plate (10) having an inner side (10a) facing towards the reaction chamber (25), and an outer side (10b) which faces away from the reaction chamber (25) and which has a plurality of connectors (11),
- providing a dimensionally stable container (20),
- providing a mixer (30), and
- providing two or more baffles, each baffle (40) having an inner fluid compartment (50) and having first and second, opposite ends, wherein the first end is adjacent the head plate and the second end is extending into the reaction chamber, each baffle further having an inlet and an outlet adapted to direct fluid flow within or through the inner fluid compartment from the inlet to the outlet, wherein the inlet and the outlet are located at the first end of the baffle,
- connecting the head plate (10) and the container (20) to each other such that they enclose a reaction chamber (25) with the mixer (30) positioned in the reaction chamber (25), and such that the at least one baffle (40) extends from the head plate (10) into the reaction chamber (25).

13. A process for operating at least one single-use bioreactor (1) according to at least one of the preceding claims 1-10,
comprising the steps:
- providing at least one single-use bioreactor (1) according to at least one of the preceding claims 1-10,
- providing a fluid reservoir, a fluid pump and a temperature control unit,
- connecting the fluid reservoir to the inner fluid compartments (50) of the baffles (40),
- supplying fluid from the fluid reservoir to the inner fluid compartments (50) of the baffles (40).

14. Use of a baffle (40) having an inner fluid compartment (50) in a single-use bioreactor (1) according to at least one of the preceding claims 1-10.

## Patentansprüche

1. Einweg-Bioreaktor (1), insbesondere geeignet zur Verwendung in einem Bioreaktorsystem, zum Züchten von Biomassepartikeln,
umfassend eines Kopfplatte, einen formstabilen Behälter (20) und einen Mischer (30), wobei die Kopfplatte (10) und der Behälter (20) einen Reaktionsraum (25) umschließen und der Mischer (30) im Reaktionsraum (25) positioniert ist,
wobei die Kopfplatte (10) eine dem Reaktionsraum (25) zugewandte Innenseite (10a) und eine von dem Reaktionsraum (25) abgewandte Außenseite (10b) aufweist, und eine Vielzahl von Verbindern (11) aufweist, und
umfassend zwei oder mehr Ablenkelemente, wobei sich jedes Ablenkelement (40) von der Kopfplatte (10) in den Reaktionsraum (25) erstreckt und jedes Ablenkelement (40) eine innere Fluidkammer (50) aufweist,
wobei jedes Ablenkelement erste und zweite, gegenüberliegende Enden aufweist, wobei das erste Ende an die Kopfplatte angrenzt und das zweite Ende sich in den Reaktionsraum erstreckt,
wobei jedes Ablenkelement einen Einlass und einen Auslass aufweist, die ausgebildet sind, eine Fluidströmung in der oder durch die innere Fluidkammer vom Einlass zum Auslass leiten,
wobei sich für jedes Ablenkelement der Einlass und der Auslass am ersten Ende des Ablenkelements befinden.

2. Einweg-Bioreaktor (1) nach mindestens einem der vorhergehenden Ansprüche, wobei der Querschnitt des Ablenkelements (40) an seinem ersten Ende (41) an der Innenseite (10a) der Kopfplatte (10) größer ist als an seinem zweiten Ende (42).

3. Einweg-Bioreaktor (1) nach mindestens einem der vorhergehenden Ansprüche, mit einer Mehrzahl von Ablenkelementen (40), vorzugsweise vier Ablenkelementen (40), die vorzugsweise in Umfangsrichtung äquidistant angeordnet sind.

4. Einweg-Bioreaktor (1) nach mindestens einem der vorhergehenden Ansprüche, wobei die Vielzahl von Ablenkelementen (40) vorzugsweise so angeordnet ist, dass sie eine Fluidströmung ermöglichen
- in Reihe durch die Vielzahl von Ablenkelementen (40), oder
- parallel durch die Vielzahl von Ablenkelementen (40), oder
wobei zwei oder mehr Ablenkelemente (40) angeordnet sind, um eine Gruppe zu bilden, in der die Ablenkelemente (40) für eine Fluidströmung in Reihe durch die Ablenkelemente (40) der Gruppe angeordnet sind, und wobei zwei oder mehr solcher Gruppen für eine Fluidströmung parallel durch die zwei oder mehr solcher Gruppen angeordnet sind.

5. Einweg-Bioreaktor (1) nach mindestens einem der vorhergehenden Ansprüche, wobei das Ablenkelement (40) so angeordnet ist, dass zwischen dem Ablenkelement (40) und einer Innenwand des Behälters (20) ein Zwischenraum (26) besteht, wobei dieser Zwischenraum (26) vorzugsweise gleich oder größer als 1/100 des Innendurchmessers des Behälters (20) ist, insbesondere gleich oder größer als 1/50 des Innendurchmessers des Behälters (20).

6. Einweg-Bioreaktor (1) nach mindestens einem der vorhergehenden Ansprüche, wobei eine Außenwand des Ablenkelements (40), insbesondere ein Abschnitt der Außenwand des Ablenkelements (40), der sich über einen maximalen Füllstand des Reaktionsraums (25) hinaus erstreckt, einen wärmeleitenden Zusatz aufweist, oder vorzugsweise aus einem Verbundmaterial besteht, das eine Kunststoffmatrix und ein wärmeleitendes Material umfasst, oder ein solches Verbundmaterial enthält.

7. Einweg-Bioreaktor (1) nach mindestens einem der vorhergehenden Ansprüche, wobei ein Einlassrohr (70) innerhalb der Fluidkammer positioniert ist, das sich von einem Einlass zu einem zweiten Ende (42) des Ablenkelements (40) erstreckt.

8. Einweg-Bioreaktor (1) nach mindestens einem der vorhergehenden Ansprüche, wobei das Ablenkelement (40) mindestens ein Befestigungselement zur Befestigung eines Abstandshalters und/oder eines Fluidverbindungselements, vorzugsweise eines Abstandshalterrings und/oder eines Fluidverbindungsrings, daran aufweist.

9. Einweg-Bioreaktor (1) nach mindestens einem der vorhergehenden Ansprüche, wobei der Einlass (61) und/oder der Auslass (62) des Ablenkelements (40) mit einem Fluidreservoir und/oder dem Auslass (62) und/oder dem Einlass (61) eines weiteren Ablenkelements (40) verbunden sind über eine Verrohrung an der Außenseite (10b) der Kopfplatte (10), über eine Verrohrung an der Innenseite (10a) der Kopfplatte (10), über einen Verbindungsring, der benachbart zur Innenseite (10a) der Kopfplatte (10) oder unterhalb eines maximalen Füllstands im Reaktionsraum (25) angeordnet ist, oder über eine Verrohrung und/oder einen Verbindungsring, die/der innerhalb der Kopfplatte (10) ausgebildet ist.

10. Einweg-Bioreaktor (1) nach mindestens einem der vorhergehenden Ansprüche, wobei das Ablenkelement (40), insbesondere ein Abschnitt des Ablenkelements (40), der sich über einen maximalen Füllstand des Reaktionsraums (25) hinaus erstreckt, einen vorstehenden Rand aufweist.

11. Biotechnologische Vorrichtung, umfassend einen Einweg-Bioreaktor (1) nach mindestens einem der vorhergehenden Ansprüche 1-10, ferner umfassend ein Fluidreservoir, eine Fluidpumpe und eine Temperaturkontrolleinheit.

12. Verfahren zur Herstellung eines Einweg-Bioreaktors (1), insbesondere geeignet zur Verwendung in einem Bioreaktorsystem, zum Züchten von Biomassepartikeln, vorzugsweise eines Einweg-Bioreaktors (1) nach mindestens einem der vorhergehenden Ansprüche 1-10,
umfassend die Schritte:
- Bereitstellen einer Kopfplatte (10), wobei die Kopfplatte (10) eine dem Reaktionsraum (25) zugewandte Innenseite (10a) und eine von dem Reaktionsraum (25) abgewandte Außenseite (10b) aufweist, und eine Vielzahl von Verbindern (11) aufweist,
- Bereitstellung eines formstabilen Behälters (20),
- Bereitstellen eines Mischers (30), und
- Bereitstellen von zwei oder mehr Ablenkelementen, wobei jedes Ablenkelement (40) eine innere Fluidkammer (50) und erste und zweite, gegenüberliegende Enden aufweist, wobei das erste Ende an die Kopfplatte angrenzt und das zweite Ende sich in den Reaktionsraum erstreckt, wobei jedes Ablenkelement ferner einen Einlass und einen Auslass aufweist, die ausgebildet sind, eine Fluidströmung in der oder durch die die innere Fluidkammer vom Einlass zum Auslass leiten, wobei der Einlass und der Auslass an dem ersten Ende des Ablenkelements angeordnet sind,
- Verbinden der Kopfplatte (10) und des Behälters (20) miteinander, so dass sie einen Reaktionsraum (25) umschließen und der Mischer (30) in dem Reaktionsraum (25) positioniert ist, und so dass sich das mindestens eine Ablenkelement (40) von der Kopfplatte (10) in den Reaktionsraum (25) erstreckt.

13. Verfahren zum Betreiben mindestens eines Einweg-Bioreaktors (1) nach mindestens einem der vorhergehenden Ansprüche 1-10,
umfassend die Schritte:
- Bereitstellen mindestens eines Einweg-Bioreaktors (1) nach mindestens einem der vorhergehenden Ansprüche 1-10,
- Bereitstellen eines Fluidreservoirs, einer Fluidpumpe und einer Temperaturkontrolleinheit,
- Verbinden des Fluidreservoirs mit den inneren Fluidkammern (50) der Ablenkelemente (40),
- Zuführen von Fluid aus dem Fluidreservoir zu den inneren Fluidkammern (50) der Ablenkelemente (40).

14. Verwendung eines Ablenkelements (40) mit einer inneren Fluidkammer (50) in einem Einweg-Bioreaktor (1) nach mindestens einem der vorangehenden Ansprüche 1-10.

## Revendications

1. Bioréacteur à usage unique (1), approprié en particulier pour une utilisation dans un système de bioréacteur, pour la croissance de particules de biomasse,
comprenant une plaque de tête, un récipient (20) dimensionnellement stable et un mélangeur (30),
dans lequel la plaque de tête (10) et le récipient (20) renferment une chambre de réaction (25) avec le mélangeur (30) positionné dans la chambre de réaction (25),
la plaque de tête (10) ayant un côté interne (10a) tourné vers la chambre de réaction (25), et un côté externe (10b) tourné à l'opposé de la chambre de réaction (25) et qui présente une pluralité de connecteurs (11), et
comprenant deux déflecteurs ou plus, chaque déflecteur (40) s'étendant à partir de la plaque de tête (10) dans la chambre de réaction (25), et chaque déflecteur (40) ayant un compartiment de fluide interne (50),
dans lequel chaque déflecteur possède des première et seconde extrémités opposées, dans lequel la première extrémité est adjacente à la plaque de tête et la seconde extrémité s'étend dans la chambre de réaction,
dans lequel chaque déflecteur présente une entrée et une sortie adaptées pour diriger un écoulement de fluide dans ou à travers le compartiment de fluide interne depuis l'entrée vers la sortie,
dans lequel pour chaque déflecteur, l'entrée et la sortie sont situées au niveau de la première extrémité du déflecteur.

2. Bioréacteur à usage unique (1) selon au moins l'une des revendications précédentes, dans lequel la section transversale du déflecteur (40) au niveau de sa première extrémité (41) du côté interne (10a) de la plaque de tête (10) est plus grande qu'au niveau de sa seconde extrémité (42).

3. Bioréacteur à usage unique (1) selon au moins l'une des revendications précédentes, comprenant une pluralité de déflecteurs (40), de préférence quatre déflecteurs (40), qui sont de préférence agencés à équidistance de manière circonférentielle.

4. Bioréacteur à usage unique (1) selon au moins l'une des revendications précédentes, dans lequel la pluralité de déflecteurs (40) est de préférence agencée pour permettre un écoulement de fluide
- en série à travers la pluralité de déflecteurs (40), ou
- en parallèle à travers la pluralité de déflecteurs (40), ou
dans lequel deux déflecteurs (40) ou plus sont agencés pour former un groupe dans lequel les déflecteurs (40) sont agencés pour un écoulement de fluide en série à travers les déflecteurs (40) du groupe, et dans lequel deux ou plus de ces groupes sont agencés pour un écoulement de fluide en parallèle à travers les deux ou plus de ces groupes.

5. Bioréacteur à usage unique (1) selon au moins l'une des revendications précédentes, dans lequel le déflecteur (40) est agencé de telle sorte qu'un espace (26) existe entre le déflecteur (40) et une paroi interne du récipient (20), dans lequel cet espace (26) est de préférence supérieur ou égal à 1/100 du diamètre interne du récipient (20), en particulier supérieur ou égal à 1/50 du diamètre interne du récipient (20).

6. Bioréacteur à usage unique (1) selon au moins l'une des revendications précédentes, dans lequel une paroi externe du déflecteur (40), en particulier une section de la paroi externe du déflecteur (40) s'étendant au-delà d'un niveau de remplissage maximal de la chambre de réaction (25) comprend un additif conducteur thermique, ou est de préférence constituée d'un matériau composite comprenant une matrice plastique et un matériau conducteur thermique, ou comprend un tel matériau composite.

7. Bioréacteur à usage unique (1) selon au moins l'une des revendications précédentes, dans lequel un tube d'entrée (70) est positionné à l'intérieur du compartiment de fluide s'étendant à partir d'une entrée vers une seconde extrémité (42) du déflecteur (40).

8. Bioréacteur à usage unique (1) selon au moins l'une des revendications précédentes, dans lequel le déflecteur (40) comprend au moins un élément de montage pour monter un espaceur et/ou un élément de connexion de fluide, de préférence une bague d'espacement et/ou une bague de connexion de fluide, sur celui-ci.

9. Bioréacteur à usage unique (1) selon au moins l'une des revendications précédentes, dans lequel l'entrée (61) et/ou la sortie (62) du déflecteur (40) sont connectées à un réservoir de fluide et/ou à la sortie (62) et/ou l'entrée (61) d'un autre déflecteur (40) via un tube du côté externe (10b) de la plaque de tête (10), via un tube du côté interne (10a) de la plaque de tête (10), via une bague de connexion positionnée de manière adjacente au côté interne (10a) de la plaque de tête (10) ou en dessous d'un niveau de remplissage maximum dans la chambre de réaction (25), ou via un tube et/ou une bague de connexion formée à l'intérieur de la plaque de tête (10).

10. Bioréacteur à usage unique (1) selon au moins l'une des revendications précédentes, dans lequel le déflecteur (40) comprend, en particulier une section du déflecteur (40) s'étendant au-delà d'un niveau de remplissage maximal de la chambre de réaction (25), un bord en saillie.

11. Dispositif biotechnologique comprenant un bioréacteur à usage unique (1) selon au moins l'une des revendications 1 à 10 précédentes, comprenant en outre un réservoir de fluide, une pompe de fluide et une unité de régulation de température.

12. Procédé de fabrication d'un bioréacteur à usage unique (1), approprié en particulier pour une utilisation dans un système de bioréacteur, pour la croissance de particules de biomasse, de préférence un bioréacteur à usage unique (1) selon au moins l'une des revendications 1 à 10 précédentes, comprenant les étapes suivantes :
- la fourniture d'une plaque de tête (10), la plaque de tête (10) ayant un côté interne (10a) tourné vers la chambre de réaction (25), et un côté externe (10b) tourné à l'opposé à la chambre de réaction (25) et qui présente une pluralité de connecteurs (11),
- la fourniture d'un récipient (20) dimensionnellement stable,
- la fourniture d'un mélangeur (30), et
- la fourniture de deux déflecteurs ou plus, chaque déflecteur (40) ayant un compartiment de fluide interne (50) et possédant des première et seconde extrémités opposées, dans lequel la première extrémité est adjacente à la plaque de tête et la seconde extrémité s'étend dans la chambre de réaction, chaque déflecteur présentant en outre une entrée et une sortie adaptées pour diriger un écoulement de fluide dans ou à travers le compartiment de fluide interne depuis l'entrée vers la sortie, dans lequel l'entrée et la sortie sont situées au niveau de la première extrémité du déflecteur,
- la connexion de la plaque de tête (10) et du récipient (20) l'un à l'autre de telle sorte qu'ils renferment une chambre de réaction (25) avec le mélangeur (30) positionné dans la chambre de réaction (25), et de telle sorte que l'au moins un déflecteur (40) s'étende à partir de la plaque de tête (10) dans la chambre de réaction (25).

13. Procédé pour faire fonctionner au moins un bioréacteur à usage unique (1) selon au moins l'une des revendications 1 à 10 précédentes,
comprenant les étapes suivantes :
- la fourniture d'au moins un bioréacteur à usage unique (1) selon au moins l'une des revendications 1 à 10 précédentes,
- la fourniture d'un réservoir de fluide, d'une pompe de fluide et d'une unité de régulation de température,
- la connexion du réservoir de fluide aux compartiments de fluide internes (50) des déflecteurs (40),
- l'introduction d'un fluide provenant du réservoir de fluide dans les compartiments de fluide internes (50) des déflecteurs (40).

14. Utilisation d'un déflecteur (40) ayant un compartiment de fluide interne (50) dans un bioréacteur à usage unique (1) selon au moins l'une des revendications 1 à 10 précédentes.
